# EUROPÄISCHE PATENTANMELDUNG

(11) **EP 0 921 113 A1**
(43) Veröffentlichungstag der Anmeldung: **09.06.1999**
(21) Anmeldenummer: 98123032.9
(22) Anmeldetag: 07.12.1998
(51) Int. Cl.: C07C 51/43, C07C 57/04

(54) **Verfahren zur Kristallisation von Methacrylsäure**

(30) Priorität: 05.12.1997 DE 19754014
(71) Anmelder: BASF AKTIENGESELLSCHAFT, 67056 Ludwigshafen (DE)
(72) Erfinder: Eck, Bernd, 68519 Viernheim (DE); Martin, Friedrich-Georg, Dr., 69124 Heidelberg (DE); Meckl, Stefan, Dr., 67157 Wachenheim (DE); Schraut, Armin, Dr., 64625 Bensheim (DE); Ulbrich, Michael-Dieter, Dr., 67251 Freinsheim (DE)
(74) Vertreter: Isenbruck, Günter, Dr.

(57) **Zusammenfassung**

In einem Verfahren zur Kristallisation von Methacrylsäure aus wasserhaltigen Lösungen, die 13 bis 70 Gew.-% Methacrylsäure bezogen auf 100 Gew.-% Methacrylsäure und Wasser enthalten, führt man die Kristallisation in Anwesenheit von Essigsäure durch.

## Beschreibung

Die vorliegende Erfindung betrifft ein Verfahren zur Kristallisation von Methacrylsäure aus wasserhaltigen Lösungen.

Zur Abtrennung und Reindarstellung von Methacrylsäure sind im Stand der Technik zahlreiche Verfahren bekannt, wie Extraktion und Kristallisation. So beschreibt EP-A-0 023 774 ein Verfahren zur Abtrennung von Methacrylsäure aus einer wäßrigen Methacrylsäure-Lösung durch Lösungsmittelextraktion, wobei die wäßrige Methacrylsäure-Lösung durch katalytische Gasphasenoxidation von Isobuten, tert.-Butanol, Methacrolein oder Isobutyraldehyd mit anschließender Absorption des Reaktionsprodukts in Wasser erhalten worden ist. T.N. Obmelyukhina et. al. beschreiben in ihrem Artikel in Khimicheskaya Promyshlennost, Band 23/4, S. 209-211 (1991), die Auftrennung von wäßrigen Lösungen von Methacrylsäure mit Essigsäure durch Rektifikation. In Zhurnal Prikladnoi Khimii, Band 47/1, S. 180-183 (Januar 1974), beschreiben A.V. Samarina, V.I. Arulin und L.I. Efimov die Reinigung von Methacrylsäure durch Zonenschmelzen. US-A-4 780 568 betrifft die Abtrennung von Methacrylsäure aus ihren, im wesentlichen wasserfreien Gemischen mit Isobuttersäure durch selektive Kristallisation. Des weiteren beschreibt EP-A-0 675 100 ein Verfahren zur Herstellung von á, â-ungesättigten C₃-C₆-Carbonsäuren, z.B. Methacrylsäure, durch oxidative Dehydrierung der entsprechenden gesättigten C₃-C₆-Carbonsäure, gefolgt von Schmelzkristallisation des bei der Dehydrierung entstehenden Reaktionsproduktes mit anschließender fraktionierter Destillation oder gefolgt von fraktionierter Destillation des bei der Dehydrierung entstehenden Reaktionsgemisches mit anschließender Schmelzkristallisation.

Es wurde überraschenderweise gefunden, daß das Phasengleichgewicht zwischen Methacrylsäure und Wasser eine Mischungslücke, d.h. zwei flüssige Phasen, über einen weiten Konzentrationsbereich zeigt. Das Fest-Flüssig-Phasengleichgewicht zwischen Methacrylsäure und Wasser ist bei D.A. Chubarov, S.M. Danov und G.V. Brovkina in Zhurnal Prikladnoi Khimii, Band 51/8, S. 1899-1900 (August 1978) beschrieben. Dabei wird auf eine vermutete Mischungslücke hingewiesen. Messungen hierzu wurden jedoch nicht durchgeführt. Das Problem einer Kristallisation von Methacrylsäure aus wasserhaltigen Lösungen besteht nun darin, daß im Bereich der Mischungslücke eine Mischung nicht abgekühlt werden kann, ohne daß sich zwei flüssige Phasen bilden. Die Bildung einer zweiten flüssigen Phase ist bei der Kristallisation aber nicht erwünscht, da dies zu Problemen bei der Durchmischung der an der Kristallisation beteiligten Phasen führt.

Im Hinblick darauf bestand die Aufgabe der vorliegenden Erfindung darin, ein Verfahren zu schaffen, bei dem bei der Kristallisation von Methacrylsäure aus wasserhaltigen Lösungen der Bereich, in dem zwei flüssige Phasen auftreten, ganz unterdrückt oder zumindest verkleinert wird.

Überraschenderweise wurde gefunden, daß Essigsäure die Mischungslücke zwischen Wasser und Methacrylsäure verkleinert beziehungsweise ganz unterdrückt.

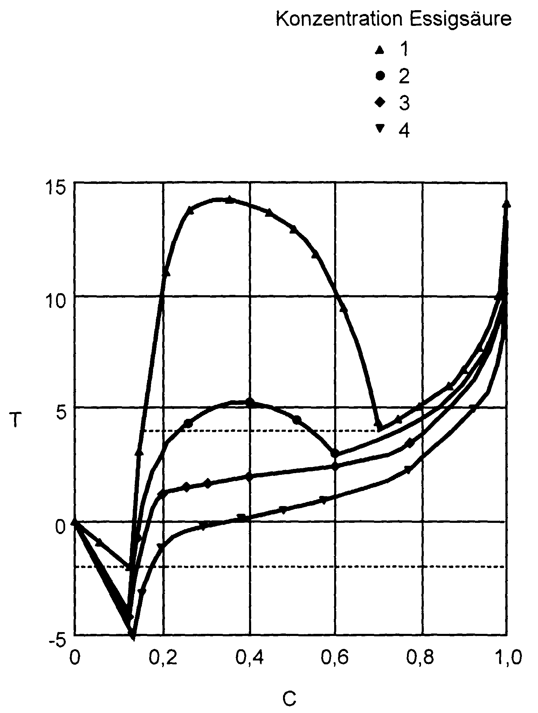

Somit betrifft die Erfindung ein Verfahren zur Kristallisation von Methacrylsäure aus wasserhaltigen Lösungen, das dadurch gekennzeichnet ist, daß man eine Lösung mit 13 bis 70 Gew.-% Methacrylsäure, bezogen auf 100 Gew.-% Methacrylsäure und Wasser, einsetzt und die Kristallisation in Anwesenheit von Essigsäure durchführt. Bevorzugte Ausführungsformen der Erfindung sind in der nachfolgenden Beschreibung, den Unteransprüchen, den Beispielen und der Figur beschrieben.

Die einzige Figur zeigt graphisch die experimentell ermittelten Meßwerte bei der Bestimmung des Fest-Flüssig- und Flüssig-Flüssig-Phasengleichgewichtes zwischen Methacrylsäure und Wasser in Abhängigkeit von der Temperatur und dem Gewichtsanteil von Methacrylsäure zu Wasser ohne und mit Essigsäure.

Geeignete Lösungen, aus denen erfindungsgemäß die Methacrylsäure auskristallisiert werden kann, enthalten 13 bis 70 Gew.-%, insbesondere 15 bis 68 Gew.-%, stärker bevorzugt 30 bis 68 Gew.-%, am meisten bevorzugt 50 bis 65 Gew.-% Methacrylsäure, und 30 bis 87 Gew.-%, insbesondere 32 bis 85 Gew.-%, stärker bevorzugt 32 bis 70 Gew.-%, am meisten bevorzugt 35 bis 50 Gew.-% Wasser, jeweils bezogen auf 100 Gew.-% Methacrylsäure und Wasser, und wenigstens 1,0 Gewichtsteil, insbesondere 1,0 bis 10 Gewichtsteile, vorzugsweise 4,0 bis 8,0 Gewichtsteile, am meisten bevorzugt 4,5 bis 6 Gewichtsteile Essigsäure, jeweils bezogen auf 100 Gewichtsteile Methacrylsäure und Wasser. Es kann auch mit weniger als 1,0 Gewichtsteil Essigsäure, bezogen auf 100 Gewichtsteile Methacrylsäure und Wasser, in der zu kristallisierenden Lösung gearbeitet werden, jedoch wird dann nicht der ganze erfindungsgemäße Vorteil erzielt, da die Mischungslücke noch nicht verschwindet, sondern in Abhängigkeit von der Essigsäurekonzentration nur verkleinert wird.

Neben der Methacrylsäure, dem Wasser und der Essigsäure kann die zu kristallisierende Lösung auch weitere Verbindungen oder Komponenten enthalten, z.B. Verunreinigungen oder Nebenkomponenten, die aus dem Herstellungsprozeß der Methacrylsäure stammen. Entsteht bei der Herstellung der Methacrylsäure bereits Essigsäure und ist diese somit von vorneherein in der zu kristallisierenden Lösung enthalten, so wird nur mehr so viel Essigsäure zugegeben, daß diese erfindungsgemäß in geeigneter Konzentration vorliegt. Eine besonders geeignete Lösung liegt vor, wenn man ein Reaktionsgasgemisch, wie es bei der katalytischen Gasphasenoxidation von C₄-Alkanen, -Alkenen, -Alkanalen und/oder -Alkanolen und/oder Vorstufen davon, z.B. Isobutan, Isobuten, Isobutyraldehyd, Methacrolein, Isobuttersäure oder Methyl-tert.-butylether entsteht, in eine Lösung überführt, z.B. durch Kondensation oder Absorption in Wasser. Die bei der katalytischen Gasphasenoxidation zu Methacrylsäure entstehenden Verunreinigungen stören also nicht bei der erfindungsgemäßen Kristallisation von Methacrylsäure. Liegen Nebenkomponenten in der zu kristallisierenden Lösung vor, dann beträgt deren Menge vorzugsweise nicht mehr als 10 Gewichtsteile, insbesondere beträgt sie 0,1 bis 5 Gewichtsteile, stärker bevorzugt 0,1 bis 3 Gewichtsteile, jeweils bezogen auf 100 Gewichtsteile Methacrylsäure und Wasser.

Die Figur zeigt graphisch die Meßergebnisse, die bei der experimentellen Bestimmung des Fest-Flüssig- und Flüssig-Flüssig-Phasengleichgewichtes von Wasser und Methacrylsäure in Abhängigkeit von der Temperatur und dem Gewichtsanteil von Methacrylsäure zu Wasser ohne und mit verschiedenen Konzentrationen an Essigsäure erhalten wurden. Hierbei beziehen sich die vier Kurven auf 0 Gewichtsteile Essigsäure (Kurve (1)), 2 Gewichtsteile Essigsäure (Kurve (2)), 4,2 Gewichtsteile Essigsäure (Kurve (3)) und 6,4 Gewichtsteile Essigsäure (Kurve (4)), wobei die Gewichtsteile Essigsäure jeweils auf 100 Gewichtsteile Methacrylsäure und Wasser bezogen sind. In der Figur bezeichnet T die Temperatur in °C und C den Anteil von Methacrylsäure zu Wasser in Gew.-% Methacrylsäure bezogen auf 100 Gew.-% Methacrylsäure und Wasser. Tabelle 1 zeigt die wesentlichen Meßergebnisse:

**Tabelle 1**

| **Essigsäuregehalt in Gewichtsteilen, bezogen auf 100 Gewichtsteile Methacrylsäure und Wasser** | **Kritische Mischungstemp. in °C** | **Größte Breite der Mischungslücke von ... bis ... Gew.-% Methacrylsäure, bezogen auf 100 Gew.-% Methacrylsäure und Wasser** |
|---|---|---|
| 0 | 14,1 | 15 bis 68 |
| 2 | 5,3 | 19 bis 56 |
| 4,2 | 2,3 | 36 bis 39 |

Wie aus der Figur und Tabelle 1 ersichtlich, liegt die kritische Entmischungstemperatur des Systems Wasser und Methacrylsäure bei 14,1 °C, d.h., bei einer Temperatur über 14,1 °C verschwindet die Mischungslücke. Die größte Breite der Mischungslücke liegt (bei 0 Gew.-% Essigsäure) bei 15 bis 68 Gew.-% Methacrylsäure. Setzt man einer zu kristallisierenden Lösung 2 Gewichtsteile Essigsäure zu, so wird die Mischungslücke auf 19 bis 56 Gew.-% Methacrylsäure verkleinert, während gleichzeitig die kritische Entmischungstemperatur auf 5,3 °C absinkt. Bei höheren Essigsäuregehalten sinkt die kritische Entmischungstemperatur weiter und die Mischungslücke wird kleiner. Bei Essigsäuregehalten über 4,5 Gewichtsteilen, bezogen auf 100 Gewichtsteile Methacrylsäure und Wasser, verschwindet die Mischungslücke dann ganz.

Das verwendete Kristallisationsverfahren unterliegt keiner Beschränkung. Die Kristallisation kann kontinuierlich oder diskontinuierlich, einstufig oder mehrstufig durchgeführt werden. Vorzugsweise erfolgt die Kristallisation einstufig. In einer anderen bevorzugten Ausführungsform der Erfindung wird die Kristallisation als fraktionierte Kristallisation durchgeführt. Üblicherweise werden bei fraktionierter Kristallisation alle Stufen, die ein Kristallisat erzeugen, das reiner ist als die zugeführte wasserhaltige Methacrylsäurelösung, Reinigungsstufen genannt und alle anderen Stufen werden Abtriebsstufen genannt. Zweckmäßigerweise werden mehrstufige Verfahren hierbei nach dem Gegenstromprinzip betrieben, bei dem nach der Kristallisation in jeder Stufe das Kristallisat von der Mutterlauge abgetrennt wird und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

Vorteilhafterweise liegt die Temperatur der Lösung während der Kristallisation zwischen +14 und -5 °C, insbesondere zwischen +5 und -5 °C. Der Feststoffgehalt im Kristallisator liegt vorteilhafterweise zwischen 0 und 80 g/100 g Suspension, bevorzugt zwischen 20 und 70 g Feststoff/100 g Suspension.

In einer vorteilhaften Ausgestaltung der Erfindung erfolgt die Kristallisation durch Kühlung von Apparatewänden oder durch Verdampfung der Lösung im Vakuum. Bei der Kristallisation durch Kühlung wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet. Daneben besteht auch die Möglichkeit, die Wärme über die Wand eines Rührkessels mit wandgängigem Rührer abzuführen. Eine weitere bevorzugte Ausführungsform bei der Kühlungskristallisation ist die Verwendung von Kühlscheibenkristallern, wie sie z.B. von der Fa. Gouda (Holland) hergestellt werden. Bei einer weiteren geeigneten Variante zur Kristallisation durch Kühlung wird die Wärme über herkömmliche Wärmeüberträger (bevorzugt Rohrbündel- oder Plattenwärmeüberträger) abgeführt. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparats wird der erste Apparat regeneriert (vorzugsweise durch Abschmelzen der Kristallschicht oder Durchspülen des Apparats mit ungesättigter Lösung). Wird im zweiten Apparat ein zu hoher Wärmedurchgangswiderstand erreicht, schaltet man wieder auf den ersten Apparat um usw. Diese Variante kann auch mit mehr als zwei Apparaten im Wechsel betrieben werden. Außerdem kann die Kristallisation durch eine herkömmliche Verdampfung der Lösung im Vakuum erfolgen. In einer weiteren vorteilhaften Ausführung der Erfindung erfolgt die Kristallisation in Apparaten, in denen die Kristalle im Kristallisationsapparat an gekühlten Flächen aufwachsen, d.h. im Apparat fixiert sind (z.B. Schichtkristallisationsverfahren der Fa. Sulzer Chemtech (Schweiz) oder Statisches Kristallisationsverfahren der Fa. BEFS PROKEM (Frankreich)).

Nach der Kristallisation werden die Methacrylsäurekristalle von der Mutterlauge abgetrennt. Für den Fall der Schichtkristallisation oder der Statischen Kristallisation kann die Trennung der Kristalle von der Mutterlauge im Kristallisationsapparat selbst erfolgen, da die Kristalle im Apparat fixiert sind und die Mutterlauge durch Abfließenlassen aus dem Apparat entfernt werden kann. Die Entfernung der Kristalle aus dem Kristallisationsapparat erfolgt durch Aufschmelzen der Kristalle und nachfolgendes Abfließenlassen der Schmelze. Für den Fall der Suspensionskristallisation eignen sich alle bekannten Verfahren der Fest-Flüssig-Trennung. In einer bevorzugten Ausführungsform der Erfindung werden die Kristalle durch Filtrieren und/oder Zentrifugieren von der Mutterlauge abgetrennt. Vorteilhafterweise wird dem Filtrieren oder Zentrifugieren eine Voreindickung der Suspension, zum Beispiel durch Hydrozyklon(e), vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk, oder mittels Bandfilter betrieben werden. Allgemein kann das Filtrieren unter Druck oder im Vakuum erfolgen.

Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/oder Schwitzen der Kristalle oder des Kristallkuchens an. Beim Waschen liegt die Waschflüssigkeitsmenge geeigneterweise zwischen 0 und 500 g Waschflüssigkeit/100 g Kristallisat, vorzugsweise zwischen 30 und 200 g Waschflüssigkeit/100 g Kristallisat. Die verwendete Waschflüssigkeit unterliegt keiner Einschränkung. Vorteilhafterweise wird jedoch mit Reinprodukt gewaschen, d.h. mit einer Flüssigkeit, die Methacrylsäure enthält, deren Reinheit höher ist als die des zu waschenden Kristallkuchens. Daneben ist auch eine Wäsche mit Wasser möglich. Das Waschen kann in hierfür üblichen Apparaten erfolgen. Vorteilhafterweise werden Waschkolonnen, in denen die Abtrennung der Mutterlauge und das Waschen in einem Apparat erfolgen, Zentrifugen, die ein- oder mehrstufig betrieben werden können, oder Filternutschen oder Bandfilter verwendet. Das Waschen kann auf Zentrifugen oder Bandfiltern ein- oder mehrstufig durchgeführt werden. Hierbei kann die Waschflüssigkeit im Gegenstrom zum Kristallkuchen geführt werden.

Beim Schwitzen handelt es sich um ein lokales Abschmelzen verunreinigter Bereiche. Vorteilhafterweise beträgt die Schwitzmenge zwischen 0 und 100 g abgeschmolzenes Kristallisat/100 g Kristallisat vor dem Schwitzen, vorzugsweise zwischen 5 und 35 g abgeschmolzenes Kristallisat/100 g Kristallisat. Besonders bevorzugt ist die Durchführung des Schwitzens auf Zentrifugen oder Bandfiltern. Auch die Durchführung einer Kombination aus Waschen und Schwitzen in einem Apparat kann geeignet sein.

Das erfindungsgemäße Verfahren ermöglicht somit eine Vergrößerung der Bereiche, in denen Methacrylsäure aus wasserhaltigen Lösungen auskristallisiert werden kann, ohne daß sich zwei flüssige Phasen bilden. Insbesondere war es überraschend, daß hierbei die zu kristallisierende Lösung weitere Verunreinigungen enthalten darf, ohne daß dieser Vorteil verloren geht.

Die Erfindung wird anhand der folgenden Beispiele, die eine bevorzugte Ausführungsform der Erfindung darstellen, näher erläutert.

### Beispiel 1

Eine Mischung aus 40 Gew.- % Methacrylsäure, 55,5 Gew.-% Wasser und 4,5 Gew.-% Essigsäure (Gesamtmasse 944 g) wurde in einem 1l Glasbehälter mit Wendelrührer einphasig flüssig vorgelegt und abgekühlt. Bei einer Temperatur von 1,7 °C bildeten sich Methacrylsäurekristalle, ohne daß sich zuvor eine zweite flüssige Phase bildete. Bei weiterer Abkühlung auf 0,4 °C bildeten sich weitere Kristalle, die auf einer Zentrifuge bei 2000 U/min (Zentrifugendurchmesser 250 mm) und einer Schleuderzeit von 2 min abgetrennt wurden. Die Masse der abgetrennten Methacrylsäurekristalle betrug 186 g und die Masse der Mutterlauge 758 g. Die Analyse des Kristallisats ergab einen Methacrylsäuregehalt von 94,3 Gew.-%. Der Essigsäuregehalt lag bei 0,13 Gew.-% und der Wassergehalt bei 5 Gew.-%.

### Beispiel 2

Eine Mischung aus 50,2 Gew.-% Methacrylsäure, 42,42 Gew.-% Wasser, 4,31 Gew.-% Essigsäure, 0,42 Gew.-% Acrylsäure, 0,31 Gew.-% Propionsäure und 2,34 Gew.-% Methacrolein (Gesamtmasse 887 g) wurde in einem 1l Glasbehälter mit Wendelrührer einphasig flüssig vorgelegt und abgekühlt. Bei einer Temperatur von -1,4 °C bildeten sich Methacrylsäurekristalle, ohne daß sich zuvor eine zweite flüssige Phase bildete. Bei weiterer Abkühlung auf -2,8 °C bildeten sich weitere Kristalle, die auf einer Zentrifuge bei 2000 U/min (Zentrifugendurchmesser 250 mm) und einer Schleuderzeit von 2 min abgetrennt wurden. Die Masse der abgetrennten Methacrylsäurekristalle betrug 202 g und die Masse der Mutterlauge 685 g. Die Analyse des Kristallisats ergab einen Methacrylsäuregehalt von 92,98 Gew.-%. Der Essigsäuregehalt lag bei 0,11 Gew.-%, der Wassergehalt bei 6,51 Gew.-%, der Acrylsäuregehalt bei 0,105 Gew.-%, der Propionsäuregehalt bei 0,034 Gew.-% und der Methacroleingehalt bei 0,257 Gew.-%.

Wie die Beispiele zeigen, ermöglicht Essigsäure die Kristallisation von Methacrylsäure aus wasserhaltigen Lösungen ohne Bildung einer zweiten flüssigen Phase. Dieser Vorteil wird auch bei Anwesenheit von Verunreinigungen beibehalten, wie Beispiel 2 zeigt.

## Patentansprüche

1. Verfahren zur Kristallisation von Methacrylsäure aus wasserhaltigen Lösungen, dadurch gekennzeichnet, daß eine Lösung mit 13 bis 70 Gew.- % Methacrylsäure, bezogen auf 100 Gew.-% Methacrylsäure und Wasser, eingesetzt wird und die Kristallisation in Anwesenheit von Essigsäure geführt wird.

2. Verfahren nach Anspruch 1, dadurch gekennzeichnet, daß eine Lösung mit wenigstens 1 Gewichtsteil Essigsäure, bezogen auf 100 Gewichtsteile Methacrylsäure und Wasser, eingesetzt wird.

3. Verfahren nach Anspruch 1 oder 2, dadurch gekennzeichnet, daß eine Lösung mit 32 bis 85 Gew.-% Wasser, bezogen auf 100 Gew.-% Methacrylsäure und Wasser, eingesetzt wird.

4. Verfahren nach einem der Ansprüche 1 bis 3, dadurch gekennzeichnet, daß eine Lösung mit 50 bis 65 Gew.-% Methacrylsäure, bezogen auf 100 Gew.-% Methacrylsäure und Wasser, eingesetzt wird.

5. Verfahren nach einem der vorhergehenden Ansprüche, dadurch gekennzeichnet, daß die Kristallisation bei einer Temperatur zwischen +14 und -5 °C durchgeführt wird.

6. Verfahren nach einem der vorhergehenden Ansprüche, dadurch gekennzeichnet, daß eine Lösung eingesetzt wird, die neben der Methacrylsäure, dem Wasser und der Essigsäure Nebenkomponenten in einer Menge von nicht mehr als 10 Gewichtsteilen, bezogen auf 100 Gewichtsteile Methacrylsäure und Wasser, enthält.

7. Verfahren nach einem der vorhergehenden Ansprüche, dadurch gekennzeichnet, daß eine Lösung eingesetzt wird, die neben der Methacrylsäure, dem Wasser und der Essigsäure Nebenkomponenten aus dem Herstellungsprozeß der Methacrylsäure enthält.

8. Verfahren nach einem der vorhergehenden Ansprüche, dadurch gekennzeichnet, daß die Kristallisation einstufig oder mehrstufig durchgeführt wird.

9. Verfahren nach einem der vorhergehenden Ansprüche, dadurch gekennzeichnet, daß die Wärme durch Kühlung von Apparatewänden oder durch Verdampfung der Lösung im Vakuum abgeführt wird.

10. Verfahren nach einem der vorhergehenden Ansprüche, dadurch gekennzeichnet, daß die erhaltenen Kristalle wenigstens einem Waschen und/oder Schwitzen unterzogen werden.
